# EUROPÄISCHE PATENTSCHRIFT

(11) **EP 4 628 134 B1**
(45) Veröffentlichungstag und Bekanntmachung des Hinweises auf die Patenterteilung: **22.07.2026**
(21) Anmeldenummer: 25167587.2
(22) Anmeldetag: 31.03.2025
(51) Int. Cl.: A61M 16/08, A61M 16/10

(54) **HALTERUNG ZUM BEFESTIGEN EINES GASSENSORS AN UND/ODER IN EINEM GEHÄUSE EINES BEATMUNGSGERÄTS**
HOLDER FOR FIXING A GAS SENSOR TO AND/OR IN A HOUSING OF A VENTILATOR
SUPPORT POUR FIXER UN CAPTEUR DE GAZ À ET/OU DANS UN BOÎTIER D'UN APPAREIL RESPIRATOIRE

(30) Priorität: 02.04.2024 DE 102024109225
(43) Veröffentlichungstag der Anmeldung: 08.10.2025
(73) Patentinhaber: Löwenstein Medical Technology S.A., 2557 Luxembourg (LU)
(72) Erfinder: Mehr, Fritz, 35799 Merenberg (DE)
(74) Vertreter: Löwenstein Medical IP

(56) Entgegenhaltungen:
- EP-B1- 3 374 012
- DE-A1- 102017 208 349
- DE-T5- 112012 003 304
- US-A1- 2019 099 578
- US-B2- 11 173 271

## Beschreibung

### Technisches Gebiet

Die Erfindung betrifft ein Beatmungsgerät mit einer Halterung zum Befestigen eines Gassensors an und/oder in einem Gehäuse des Beatmungsgeräts.

### Stand der Technik

Ein Beatmungsgerät ist in der Regel mit einem sogenannten Sauerstoffkalibriermodul zum Bestimmen einer Sauerstoffkonzentration im Atemgasstrom ausgestattet. Zu Wartungs-, Reinigungs- oder Reparaturzwecken sollte das Sauerstoffkalibriermodul einfach entnehmbar sein, insbesondere ohne dass dazu das Gehäuse des Beatmungsgeräts entfernt werden muss.

DE 11 2012 003304 T5 offenbart eine Halterung für ein Beatmungsgerät. Die Halterung umfasst einen Grundkörper mit einem Befestigungsabschnitt zum Befestigen des Grundkörpers an einem Schlauch des Beatmungsgeräts, einem Gaseinlass, einem Gasauslass, einer ersten Aufnahme zum Aufnehmen einer Koppeleinrichtung und einer zweiten Aufnahme zum Aufnehmen eines Gassensors zum Erfassen einer Gaskonzentration. Dabei begrenzt der Grundkörper zusammen mit dem von der zweiten Aufnahme aufgenommenen Gassensor eine Atemgaskammer, die einerseits mit dem Gaseinlass und andererseits mit dem Gasauslass verbunden ist, sodass Atemgas von einer Austrittsöffnung der von der ersten Aufnahme aufgenommenen Koppeleinrichtung über den Gaseinlass in die Atemgaskammer und von der Atemgaskammer über den Gasauslass in eine Umgebung des Grundkörpers strömen kann.

US 2019/099578 A1 offenbart einen Beatmungsadapter zur Verabreichung inhalierbarer Medien in die Lunge. Der Beatmungsadapter umfasst einen Inspirationsarm mit einem Medienanschluss und einen Exspirationsarm. Jeder Arm kann einerseits an ein Beatmungsgerät und andererseits an eine Patientenschnittstelle angeschlossen werden. Des Weiteren umfasst der Beatmungsadapter eine den Inspirationsarm mit dem Exspirationsarm fluidisch verbindende Bypass-Brücke und ein oder mehrere Ventile zur Steuerung einer Gasströmung durch den Beatmungsadapter.

### Offenbarung der Erfindung

Eine Aufgabe der Erfindung kann darin gesehen werden, ein Beatmungsgerät bereitzustellen, das eine verbesserte Montage bzw. Demontage eines Gassensors an und/oder in dem Beatmungsgerät ermöglicht.

Diese Aufgabe wird durch den Gegenstand des unabhängigen Anspruchs gelöst. Vorteilhafte Ausführungsformen der Erfindung sind in den abhängigen Ansprüchen, der nachfolgenden Beschreibung und den beigefügten Figuren dargelegt.

Offenbart wird eine Halterung zum Befestigen eines Gassensors an und/oder in einem Gehäuse eines Beatmungsgeräts. Das Beatmungsgerät kann zusätzlich zum Gassensor und zum Gehäuse einen Atemgasanschluss zum Anschließen einer Atemgasquelle, einen Patientenanschluss zum Anschließen einer Patientenschnittstelle und eine Koppeleinrichtung zum fluidischen Koppeln des Gassensors mit dem Atemgasanschluss und/oder dem Patientenanschluss umfassen. Die Halterung umfasst einen Grundkörper mit einem Befestigungsabschnitt zum Befestigen des Grundkörpers am und/oder im Gehäuse, einem Gaseinlass, einem Gasauslass, einer ersten Aufnahme zum Aufnehmen der Koppeleinrichtung, sodass eine Austrittsöffnung
der Koppeleinrichtung mit dem Gaseinlass fluidisch gekoppelt ist, und einer (beispielsweise neben der ersten Aufnahme angeordneten) zweiten Aufnahme zum Aufnehmen des Gassensors. Der Grundkörper ist so ausgebildet, dass er zusammen mit dem von der zweiten Aufnahme aufgenommenen Gassensor eine Atemgaskammer begrenzt, die einerseits mit dem Gaseinlass und andererseits mit dem Gasauslass verbunden ist, sodass - wenn die Koppeleinrichtung von der ersten Aufnahme aufgenommen ist - Atemgas von der Austrittsöffnung über den Gaseinlass in die Atemgaskammer und von der Atemgaskammer über den Gasauslass in eine Umgebung des Grundkörpers strömen kann.

Eine solche Halterung ermöglicht eine einfache Montage bzw. Demontage des Gassensors zusammen mit dem Grundkörper.

Die erste Aufnahme kann ausgebildet sein, um zumindest einen Abschnitt der Koppeleinrichtung, insbesondere zumindest einen die Austrittsöffnung aufweisenden Abschnitt, aufzunehmen, sodass der Grundkörper luftdicht mit der Koppeleinrichtung verbunden ist.

Die zweite Aufnahme kann ausgebildet sein, um zumindest einen Abschnitt des Gassensors aufzunehmen, sodass der Grundkörper luftdicht mit dem Gassensor verbunden ist. Zusätzlich oder alternativ kann die zweite Aufnahme ausgebildet sein, um den Gassensor in einer definierten Position und/oder Orientierung relativ zum Grundkörper zu fixieren. Insbesondere kann die Halterung so ausgebildet sein, dass der von der zweiten Aufnahme aufgenommene Gassensor im betriebsfähigen Zustand des Beatmungsgeräts ausschließlich oder größtenteils vom Grundkörper getragen wird.

Der Befestigungsabschnitt kann beispielsweise ein oder mehrere Löcher zum Hindurchführen einer oder mehrerer Schrauben umfassen. Somit kann der Grundkörper einfach und schnell am und/oder im Gehäuse verschraubt werden. Es ist möglich, dass der Befestigungsabschnitt eine oder mehrere Ösen und/oder Gewindeaufnahmen zum Verschrauben des Grundkörpers mit dem Gehäuse umfasst. Alternativ oder zusätzlich kann der Befestigungsabschnitt ein oder mehrere Rastelemente wie z. B. Haken oder Nasen zum Einrasten des Grundkörpers im Gehäuse umfassen.

Der Grundkörper kann beispielsweise einteilig und/oder aus ein und demselben Material gefertigt sein. Insbesondere kann der Grundkörper ein Spritzgießteil, vorzugsweise aus Kunststoff, sein.

Die Erfindung betrifft ein Beatmungsgerät. Das Beatmungsgerät umfasst: einen Atemgasanschluss zum Anschließen einer Atemgasquelle ans Beatmungsgerät; einen Patientenanschluss zum Anschließen einer Patientenschnittstelle ans Beatmungsgerät; einen Gassensor zum Erfassen einer Gaskonzentration; eine Koppeleinrichtung, die eine mit dem Atemgasanschluss verbundene erste Eintrittsöffnung, eine mit dem Patientenanschluss verbundene zweite Eintrittsöffnung und eine Austrittsöffnung umfasst, wobei die Koppeleinrichtung zwischen einer Kalibrierstellung und einer Messstellung verstellbar ist und ausgebildet ist, um die Austrittsöffnung in der Kalibrierstellung nur mit der ersten Eintrittsöffnung und in der Messstellung entweder nur mit der zweiten Eintrittsöffnung oder sowohl mit der ersten Eintrittsöffnung als auch mit der zweiten Eintrittsöffnung fluidisch zu koppeln; ein Gehäuse, das zumindest einen Teil der Komponenten des Beatmungsgeräts umgibt, wobei die vom Gehäuse umgebenen Komponenten des Beatmungsgeräts den Gassensor und/oder die Koppeleinrichtung umfassen; eine Halterung, wie sie vor- und nachstehend beschrieben wird, wobei der Grundkörper über den Befestigungsabschnitt am und/oder im Gehäuse befestigt ist, wobei die Koppeleinrichtung von der ersten Aufnahme aufgenommen ist, sodass die Austrittsöffnung mit dem Gaseinlass fluidisch gekoppelt ist, und wobei der Gassensor von der zweiten Aufnahme aufgenommen ist, sodass der Grundkörper zusammen mit dem von der zweiten Aufnahme aufgenommenen Gassensor die Atemgaskammer begrenzt.

Das Beatmungsgerät kann zur invasiven und/oder nicht invasiven Beatmung eines Patienten ausgebildet sein.

Unter "Atemgasquelle" kann beispielsweise eine Gasflasche, ein Atemgasmischer, ein Atemgasleitungssystem (z. B. in einem Klinikgebäude) oder eine Kombination aus mindestens zwei dieser Beispiele verstanden werden.

Unter "Patientenschnittstelle" kann beispielsweise eine Beatmungsmaske, ein Tubus oder eine Nasenbrille verstanden werden.

Unter "Gassensor" kann beispielsweise ein Sauerstoffsensor mit einer galvanischen Zelle oder ein paramagnetischer Sauerstoffsensor verstanden werden. Möglich ist auch ein optischer Gassensor und/oder ein Gassensor zum Erfassen einer Konzentration mindestens eines anderen Gases als Sauerstoff, beispielsweise Stickstoff, Kohlenstoffdioxid oder Narkosegas.

Der Gassensor kann so am Grundkörper angeordnet sein, dass das Atemgas beim Strömen durch die Atemgaskammer den Gassensor passiert, sodass der Gassensor eine Gaskonzentration des den Gassensor passierenden Atemgases erfassen kann.

Die Koppeleinrichtung kann beispielsweise ein T- oder Y-förmiges Rohrstück umfassen. In diesem Fall können die Austrittsöffnung, die erste Eintrittsöffnung und die zweite Eintrittsöffnung jeweils durch eines von drei offenen Enden des Rohrstücks gebildet sein.

Zusätzlich oder alternativ kann die Koppeleinrichtung mit einem die Austrittsöffnung aufweisenden Anschlussstutzen ausgebildet sein (siehe auch weiter unten). Der Anschlussstutzen kann in einer Einführrichtung in eine Buchse der zweiten Aufnahme des Grundkörpers einführbar sein, um die Austrittsöffnung mit dem Gaseinlass des Grundkörpers fluidisch zu koppeln. Der Anschlussstutzen kann zweckmäßigerweise so ausgebildet sein, dass er mit der Buchse luftdicht und/oder ausschließlich kraftschlüssig verbunden ist, wenn er in die Buchse eingeführt ist. Die Austrittsöffnung kann beispielsweise ein freies, offenes Ende des Anschlussstutzens sein. Optional kann der Anschlussstutzen einen oder mehrere Dichtringe zum Herstellen einer luftdichten Verbindung mit der Buchse umfassen. Beispielsweise kann sich der Dichtring in seiner Längsrichtung betrachtet oder können sich die Dichtringe in ihrer jeweiligen Längsrichtung betrachtet in einer Umfangsrichtung einer äußeren und/oder inneren Mantelfläche des Anschlussstutzens erstrecken.

Die Koppeleinrichtung kann im Inneren des Beatmungsgeräts fixiert, beispielsweise verschraubt sein. Somit verbleibt die Koppeleinrichtung im Beatmungsgerät, wenn die Halterung, gegebenenfalls zusammen mit dem Gassensor, entnommen wird.

Es ist möglich, dass der Gassensor am Grundkörper fixiert, beispielsweise damit verschraubt ist, insbesondere derart, dass der Gassensor ausschließlich oder größtenteils vom Grundkörper getragen wird.

Beispielsweise kann der Gassensor über eine Steckverbindung mit einer Steuervorrichtung zum Steuern eines Betriebs des Beatmungsgeräts zur Datenkommunikation und/oder zur Stromversorgung verbunden sein.

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform können die erste Aufnahme und die Koppeleinrichtung miteinander zu einer (luftdichten) Steckverbindung verbindbar sein. Die Steckverbindung kann zweckmäßigerweise wiederlösbar ausgebildet sein, beispielsweise durch Auseinanderziehen der ersten Aufnahme und der Koppeleinrichtung mit einer definierten Kraft. Beispielsweise kann die erste Aufnahme mit einer Buchse ausgebildet sein, wobei die Koppeleinrichtung mit einem zur Buchse komplementären und die Austrittsöffnung aufweisenden Anschlussstutzen ausgebildet sein kann. Alternativ oder zusätzlich kann die erste Aufnahme mit einem Anschlussstutzen ausgebildet sein, wobei die Koppeleinrichtung mit einer zum Anschlussstutzen komplementären und die Austrittsöffnung aufweisenden Buchse ausgebildet sein kann. In beiden Fällen kann die (luftdichte) Steckverbindung durch Einführen des jeweiligen Anschlussstutzens in die jeweilige Buchse herstellbar sein. Unter "Anschlussstutzen" kann allgemein ein in eine Buchse einführbarer Stecker verstanden werden.

Gemäß einer Ausführungsform kann die erste Aufnahme eine Buchse zum Einführen eines die Austrittsöffnung aufweisenden Anschlussstutzens der Koppeleinrichtung in einer Einführrichtung umfassen. Alternativ oder zusätzlich kann die erste Aufnahme einen Anschlussstutzen zum Einführen in eine die Austrittsöffnung aufweisende Buchse der Koppeleinrichtung in einer Einführrichtung umfassen. Dies ermöglicht es, den Grundkörper beim Einführen ins Gehäuse mit der Koppeleinrichtung luftdicht zu verbinden, ohne dass zusätzliche Handgriffe nötig sind.

Gemäß einer Ausführungsform kann die Buchse einen sich in der Einführrichtung verjüngenden Querschnitt haben. Auf diese Weise kann durch bloßes Einführen in der Einführrichtung eine ausreichend kraftschlüssige und dauerhaft luftdichte Verbindung zwischen der Buchse und dem Anschlussstutzen hergestellt werden. Entsprechend einfach kann die Verbindung beim Entnehmen des Grundkörpers wieder gelöst werden, indem die Buchse und der Anschlussstutzen mit einem gewissen Kraftaufwand auseinandergezogen werden. Optional kann die Buchse einen oder mehrere Dichtringe zum Herstellen einer luftdichten Verbindung mit dem Anschlussstutzen umfassen. Beispielsweise kann sich der Dichtring in seiner Längsrichtung betrachtet oder können sich die Dichtringe in ihrer jeweiligen Längsrichtung betrachtet in einer Umfangsrichtung einer äußeren und/oder inneren Mantelfläche der Buchse erstrecken.

Gemäß einer Ausführungsform kann die zweite Aufnahme ein Gewinde zum Verschrauben des Gassensors mit dem Grundkörper umfassen. Alternativ kann die zweite Aufnahme mindestens drei Gewinde zum Verschrauben des Gassensors mit dem Grundkörper umfassen. Dies ermöglicht eine sichere und einfach zu lösende Fixierung des Gassensors am Grundkörper.

Gemäß einer Ausführungsform können die Gewinde ein zentrales Gewinde und mindestens zwei um das zentrale Gewinde herum angeordnete Schraubengewinde umfassen, wobei in jedes der Schraubengewinde eine Schraube geschraubt werden kann. Zusätzlich oder alternativ kann das zentrale Gewinde ausgebildet sein, um ein Gehäusegewinde an einem zylinderförmigen Abschnitt eines Gehäuses des Gassensors, insbesondere eines Außengehäuses des Gassensors, aufzunehmen. Beispielsweise können die Gewinde so angeordnet sein, dass deren (beispielsweise zueinander parallele) Mittelachsen eine gemeinsame gerade Linie schneiden. Dabei kann das zentrale Gewinde zwischen den zwei Schraubengewinden liegen. Denkbar sind aber auch beliebige andere Anordnungen. Es ist möglich, dass das zentrale Gewinde einen deutlich größeren Durchmesser als jedes der Schraubengewinde hat und/oder die Atemgaskammer begrenzt. Anders ausgedrückt kann zumindest ein Abschnitt des zentralen Gewindes ein die Atemgaskammer begrenzender Abschnitt des Grundkörpers sein. Zusätzlich oder alternativ kann das Gehäusegewinde oder der zylinderförmige Abschnitt bzw. können das Gehäusegewinde und der zylinderförmige Abschnitt die Atemgaskammer begrenzen. Anders ausgedrückt kann zumindest ein Abschnitt des Gehäusegewindes und/oder des zylinderförmigen Abschnitts ein die Atemgaskammer begrenzender Abschnitt des Gassensors sein. Beispielsweise kann der Durchmesser des zentralen Gewindes mindestens 2-mal, mindestens 3-mal oder mindestens 4-mal so groß wie der Durchmesser eines jeden der Schraubengewinde sein.

Gemäß einer Ausführungsform kann der Grundkörper ferner einen Verbindungskanal umfassen. In diesem Fall kann der Gaseinlass über den Verbindungskanal mit der Atemgaskammer verbunden sein. Zwischen dem Verbindungskanal und der Atemgaskammer kann zusätzlich eine Engstelle angeordnet sein. Die Engstelle kann so ausgebildet sein, dass ein Strom des über die Engstelle in die Atemgaskammer eintretenden Atemgases abhängig von einem Druck des Atemgases im Verbindungskanal in einem gewünschten Wertebereich liegt. Die Engstelle kann mit einem (deutlich) kleineren Strömungsquerschnitt als der Verbindungskanal ausgebildet sein. Beispielsweise kann der Strömungsquerschnitt der Engstelle höchstens 90 %, höchstens 50 %, höchstens 10 % oder höchstens 5 % des Strömungsquerschnitts des Verbindungskanals betragen. Die Engstelle kann beispielsweise als eine der Atemgaskammer vorgeschaltete Düse ausgebildet sein.

Gemäß einer Ausführungsform kann die zweite Aufnahme einen getrennt vom restlichen Grundkörper montierbaren Adapter zum Befestigen des Gassensors am Grundkörper umfassen. Beispielsweise kann der Adapter mit dem restlichen Grundkörper und/oder dem Gassensor mittels mehrerer Schrauben verschraubbar sein. Der Adapter kann auch als Teil eines Gehäuses und/oder einer tragenden Struktur des Gassensors ausgebildet sein. Beispielsweise kann der Adapter rahmen- und/oder plattenförmig ausgebildet sein. Dies ermöglicht es, wahlweise verschiedene Typen des Gassensors am Grundkörper zu befestigen, ohne dass der Grundkörper oder die zweite Aufnahme eigens an den jeweiligen Typ angepasst werden muss.

Gemäß einer Ausführungsform kann der Grundkörper einen Teil einer Außenwand und/oder Innenwand des Gehäuses bilden, wenn er am und/oder im Gehäuse befestigt ist.

Gemäß einer Ausführungsform kann das Gehäuse eine (durchgehende) Gehäuseöffnung zum Einführen des Gassensors ins Innere des Beatmungsgeräts aufweisen. In diesem Fall kann der Grundkörper die Gehäuseöffnung teilweise oder vollständig abdecken, wenn er am und/oder im Gehäuse befestigt ist.

Gemäß einer Ausführungsform können die erste Aufnahme und die zweite Aufnahme an einer dem Inneren des Gehäuses (oder des Beatmungsgeräts) zugewandten ersten Seite des Grundkörpers angeordnet sein. In diesem Fall kann eine der ersten Seite gegenüberliegende zweite Seite des Grundkörpers einen Teil der Außenwand des Gehäuses bilden.

Gemäß einer Ausführungsform kann die Koppeleinrichtung abhängig von einer Druckdifferenz zwischen einem an der ersten Eintrittsöffnung anliegenden ersten Atemgasdruck und einem an der zweiten Eintrittsöffnung anliegenden zweiten Atemgasdruck, beispielsweise einem Ausatmungsdruck, zwischen der Kalibrierstellung und der Messstellung verstellbar sein. Beispielsweise kann die Koppeleinrichtung ausschließlich druckabhängig verstellbar sein.

Gemäß einer Ausführungsform kann sich die Koppeleinrichtung in der Kalibrierstellung befinden, wenn der erste Atemgasdruck größer als der zweite Atemgasdruck oder gleich dem zweiten Atemgasdruck ist. Zusätzlich oder alternativ kann sich die Koppeleinrichtung in der Messstellung befinden, wenn der erste Atemgasdruck kleiner als der zweite Atemgasdruck ist. Anders ausgedrückt ist es möglich, dass sich die Koppeleinrichtung in der Kalibrierstellung befindet, wenn die Druckdifferenz ungleich null ist und ein erstes Vorzeichen hat (beispielsweise positiv ist) oder wenn die Druckdifferenz gleich null ist, und in der Messstellung befindet, wenn die Druckdifferenz ungleich null ist und ein dem ersten Vorzeichen entgegengesetztes zweites Vorzeichen hat (beispielsweise negativ ist).

Gemäß einer Ausführungsform kann die Koppeleinrichtung ferner eine Basis und ein mit der Basis verbundenes und relativ zur Basis auslenkbares Dichtelement umfassen. In diesem Fall kann das Dichtelement mittels der Basis derart in einem Strömungsweg des Atemgases durch die Koppeleinrichtung positioniert sein, dass das Dichtelement abhängig von der Druckdifferenz zwischen dem ersten Atemgasdruck und dem zweiten Atemgasdruck zwischen der Kalibrierstellung und der Messstellung relativ zur Basis ausgelenkt werden kann. Das Dichtelement kann die zweite Eintrittsöffnung in der Kalibrierstellung luftdicht verschließen und in der Messstellung freigeben. Die Kalibrierstellung kann beispielsweise einer Ruhestellung des Dichtelements entsprechen. Alternativ kann die Messstellung der Ruhestellung entsprechen. Unter "Basis" kann allgemein ein Bauteil zum Halten und/oder beweglichen Lagern des Dichtelements verstanden werden. Unter "Dichtelement" kann beispielsweise eine Membran oder ein Kolben verstanden werden. Die Basis und das Dichtelement können mehrteilig oder einteilig ausgeführt sein. Beispielsweise können die Basis und das Dichtelement in einer einteiligen Ausführung aus ein und demselben Material (z. B. Kunststoff), insbesondere als ein Spritzgießteil, gefertigt sein.

Gemäß einer Ausführungsform kann das Dichtelement derart elastisch federnd ausgebildet sein und/oder derart elastisch federnd mit der Basis verbunden sein, dass es in der Messstellung mit einer in Richtung der Kalibrierstellung wirkenden Rückstellkraft beaufschlagt wird. Auf diese Weise kann sich das Dichtelement von allein in die Kalibrierstellung zurückstellen, wenn es nicht anderweitig daran gehindert wird. Das Dichtelement kann so ausgebildet sein, dass es entweder nur in der Kalibrierstellung oder sowohl in der Kalibrierstellung als auch in der Messstellung ein Strömen des Atemgases von der ersten Eintrittsöffnung zur Austrittsöffnung ermöglicht.

Gemäß einer Ausführungsform kann das Dichtelement über mindestens zwei elastisch verformbare Federarme elastisch federnd mit der Basis verbunden sein. Die Basis, das Dichtelement und die Federarme können mehrteilig oder einteilig ausgeführt sein. Beispielsweise können die Basis, das Dichtelement und die Federarme in einer einteiligen Ausführung aus ein und demselben Material (z. B. Kunststoff), insbesondere als ein Spritzgießteil, gefertigt sein.

Gemäß einer Ausführungsform kann jeder der Federarme zwei kürzere Längsabschnitte und einen die zwei kürzeren Längsabschnitte miteinander verbindenden längeren Längsabschnitt umfassen. Der längere Längsabschnitt kann länger als jeder der zwei kürzeren Längsabschnitte oder länger als die zwei kürzeren Längsabschnitte zusammen sein. Ein erster der zwei kürzeren Längsabschnitte kann in einer von einer Längsrichtung des längeren Längsabschnitts abweichenden ersten Richtung vom längeren Längsabschnitt, beispielsweise von dessen erstem Ende, abstehen, wohingegen ein zweiter der zwei kürzeren Längsabschnitte in einer von der ersten Richtung und/oder der Längsrichtung des längeren Längsabschnitts abweichenden zweiten Richtung vom längeren Längsabschnitt, beispielsweise von dessen zweitem Ende, abstehen kann. Bei der ersten und der zweiten Richtung kann es sich beispielsweise um einander entgegengesetzte Richtungen, um zueinander orthogonale Richtungen oder um zueinander schräge Richtungen handeln. Beispielsweise kann der längere Längsabschnitt mindestens 2-mal, mindestens 4-mal oder mindestens 10-mal so lang wie jeder der zwei kürzeren Längsabschnitte oder die zwei kürzeren Längsabschnitte zusammen sein. Jeder der Federarme kann über seinen ersten kürzeren Längsabschnitt mit der Basis und über seinen zweiten kürzeren Längsabschnitt mit dem Dichtelement verbunden sein.

Gemäß einer Ausführungsform kann der längere Längsabschnitt in seiner Längsrichtung betrachtet zumindest teilweise gekrümmt verlaufen. Solche Federarme können die Federung bei gegebenem Bauraum im Vergleich zu Ausführungen mit anders geformten Federarmen effizienter und/oder haltbarer machen.

Gemäß einer Ausführungsform kann die Basis ringförmig sein und/oder kann das Dichtelement scheibenförmig sein. Zusätzlich können die ringförmige Basis und das scheibenförmige Dichtelement eine gemeinsame Mittelachse haben, d. h. konzentrisch zueinander angeordnet sein. In diesem Fall kann das Dichtelement gegenüber und/oder innerhalb einer Öffnung der Basis angeordnet sein und/oder entlang der gemeinsamen Mittelachse relativ zur Basis zwischen der Kalibrierstellung und der Messstellung auslenkbar sein.

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt ein Beatmungsgerät gemäß einer Ausführungsform der Erfindung.
Fig. 2 zeigt eine Halterung eines Beatmungsgeräts gemäß einer Ausführungsform der Erfindung, wobei der Grundkörper der Halterung mit einem Gassensor in Form einer paramagnetischen Messzelle verschraubt ist.
Fig. 3 zeigt die Halterung aus Fig. 2 ohne den Gassensor und mit eingeführter Koppeleinrichtung.
Fig. 4 zeigt einen Gassensor in Form einer chemischen Messzelle, deren Gehäuse mit dem Grundkörper der Halterung aus Fig. 3 verschraubbar ist.
Fig. 5 zeigt einen Querschnitt durch die Koppeleinrichtung aus Fig. 3.
Fig. 6 zeigt einen Querschnitt durch den Grundkörper aus Fig. 3 und den damit verschraubten Gassensor aus Fig. 4.
Fig. 7 zeigt eine Draufsicht auf eine Dichtmembran zur Verwendung in einer Koppeleinrichtung eines Beatmungsgeräts gemäß einer Ausführungsform der Erfindung.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

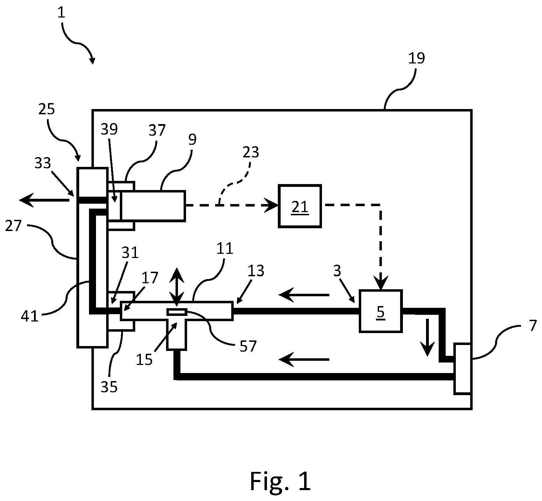

Fig. 1 zeigt Komponenten eines Beatmungsgeräts 1. Das Beatmungsgerät 1 umfasst in diesem Beispiel einen Atemgasanschluss 3 zum Anschließen einer Atemgasquelle 5 ans Beatmungsgerät 1, einen Patientenanschluss 7 zum Anschließen einer Patientenschnittstelle ans Beatmungsgerät 1, einen Gassensor 9 zum Erfassen einer Gaskonzentration sowie eine Koppeleinrichtung 11. Die Koppeleinrichtung 11 hat eine mit dem Atemgasanschluss 3 verbundene erste Eintrittsöffnung 13, eine mit dem Patientenanschluss 7 verbundene zweite Eintrittsöffnung 15 und eine Austrittsöffnung 17. Zudem ist die Koppeleinrichtung 11 zwischen einer Kalibrierstellung und einer Messstellung verstellbar und ausgebildet, um die Austrittsöffnung 17 in der Kalibrierstellung nur mit der ersten Eintrittsöffnung 13 und in der Messstellung entweder nur mit der zweiten Eintrittsöffnung 15 oder sowohl mit der ersten Eintrittsöffnung 13 als auch mit der zweiten Eintrittsöffnung 15 fluidisch zu koppeln, sodass Atemgas in der Kalibrierstellung von der ersten Eintrittsöffnung 13 und somit von der Atemgasquelle 5 (beispielsweise einer Gasflasche, einem Atemgasmischer oder einem Atemgasleitungssystem) zur Austrittsöffnung 17 strömen kann und in der Messstellung zumindest von der zweiten Eintrittsöffnung 15 und somit zumindest von der Patientenschnittstelle (beispielsweise einer Beatmungsmaske, einem Tubus oder einer Nasenbrille) zur Austrittsöffnung 17 strömen kann.

Ferner umfasst das Beatmungsgerät 1 ein Gehäuse 19, das zumindest einen Teil der Komponenten des Beatmungsgeräts 1 umgibt, hier unter anderem die Atemgasquelle 5, den Gassensor 9, die Koppeleinrichtung 11 und eine optionale Steuervorrichtung 21 zum Steuern der Atemgasquelle 5 unter Verwendung eines vom Gassensor 9 bereitgestellten Sensorsignals 23.

Darüber hinaus umfasst das Beatmungsgerät 1 eine Halterung 25, über die der Gassensor 9 am Gehäuse 19 befestigt, beispielsweise verschraubt ist, insbesondere derart, dass sich der Gassensor 9 vollständig oder größtenteils im Inneren des Gehäuses 19 befindet.

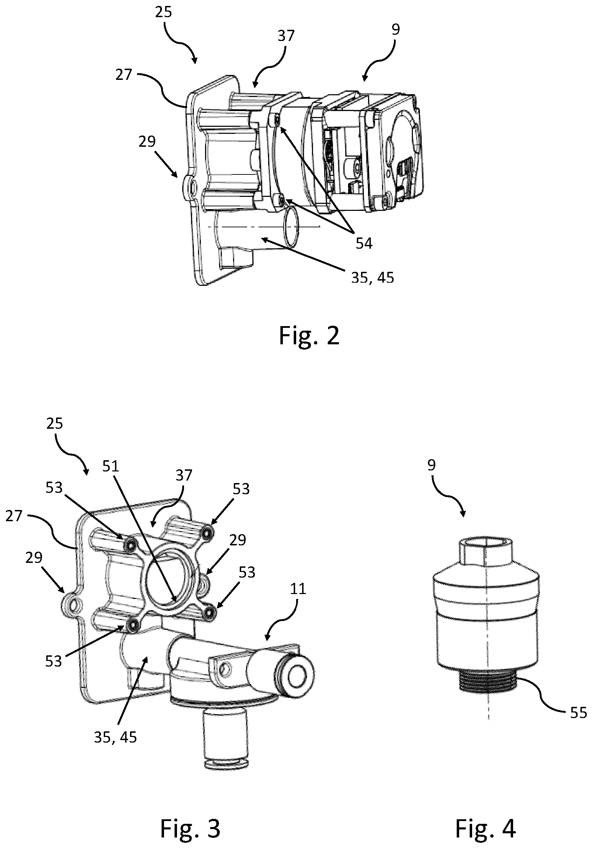

Die Halterung 25 umfasst einen Grundkörper 27 mit einem Befestigungsabschnitt 29 (siehe Fig. 2 und Fig. 3), über den der Grundkörper 27 am Gehäuse 19 befestigt, hier verschraubt ist, einem Gaseinlass 31, einem Gasauslass 33, einer ersten Aufnahme 35 und einer zweiten Aufnahme 37. In diesem Beispiel sind die beiden Aufnahmen 35, 37 derart am Grundkörper 27 angeordnet, dass sie im betriebsfähigen Zustand des Beatmungsgeräts 1, wie er in Fig. 1 gezeigt ist, übereinanderliegen. Alternativ oder zusätzlich zu unterschiedlichen vertikalen Positionen können die beiden Aufnahmen 35, 37 im betriebsfähigen Zustand des Beatmungsgeräts 1 unterschiedliche horizontale Positionen haben.

Die erste Aufnahme 35 ist ausgebildet, um zumindest einen Abschnitt der Koppeleinrichtung 11 aufzunehmen, sodass die Austrittsöffnung 17 mit dem Gaseinlass 31 fluidisch gekoppelt ist.

Die zweite Aufnahme 37 ist ausgebildet, um zumindest einen Abschnitt des Gassensors 9 aufzunehmen. Der Grundkörper 27 begrenzt zusammen mit dem von der zweiten Aufnahme 37 aufgenommenen Gassensor 9 eine Atemgaskammer 39, die einerseits mit dem Gaseinlass 31 und andererseits mit dem Gasauslass 33 verbunden ist, sodass das Atemgas von der Austrittsöffnung 17 über den Gaseinlass 31 in die Atemgaskammer 39 und von dort über den Gasauslass 33 in eine Umgebung des Grundkörpers 27 strömen kann. Beim Strömen durch die Atemgaskammer 39 passiert das Atemgas den Gassensor 9, sodass der Gassensor 9 die Gaskonzentration des den Gassensor 9 passierenden Atemgases erfassen und ein entsprechendes Sensorsignal 23 erzeugen kann.

Eine Strömungsrichtung des Atemgases durch das Beatmungsgerät 1 ist in Fig. 1 beispielhaft mit durchgehenden Pfeilen angedeutet.

Das Gehäuse 19 kann eine gesonderte Gehäuseöffnung zum Einführen des an der Halterung 25 befestigten Gassensors 9 ins Innere des Gehäuses 19 (oder ins Innere des Beatmungsgeräts 1) aufweisen. Die Gehäuseöffnung kann zweckmäßigerweise an einer Seitenwand des Gehäuses 19 ausgebildet sein. Möglich sind aber auch andere Positionen der Gehäuseöffnung, beispielsweise an einer Unter- oder Oberseite des Beatmungsgeräts 1 im betriebsfähigen Zustand. Der Grundkörper 27 kann die Gehäuseöffnung im montierten Zustand der Halterung 25 teilweise oder vollständig, insbesondere staub- und/oder fluiddicht, abdecken. Dabei kann der Grundkörper 27 einen Teil einer Außen- und/oder Innenfläche des Gehäuses 19 bilden.

Die beiden Aufnahmen 35, 37 können sich im montierten Zustand der Halterung 25 an einer dem Inneren des Gehäuses 19 (oder dem Inneren des Beatmungsgeräts 1) zugewandten ersten Seite des Grundkörpers 27 befinden. Dabei kann eine der ersten Seite gegenüberliegende zweite Seite des Grundkörpers 27 einen Teil der Außenfläche oder einer Außenwand des Gehäuses 19 bilden.

Zusätzlich kann der Grundkörper 27 einen Verbindungskanal 41 umfassen, der an seinem ersten Ende mit dem Gaseinlass 31 und an seinem zweiten Ende mit der Atemgaskammer 39 verbunden ist, sodass das Atemgas über den Verbindungskanal 41 vom Gaseinlass 31 zur Atemgaskammer 39 strömen kann. Der Verbindungskanal 41 kann beispielsweise durch eine entsprechende Ausnehmung in einem Material des Grundkörpers 27 gebildet sein.

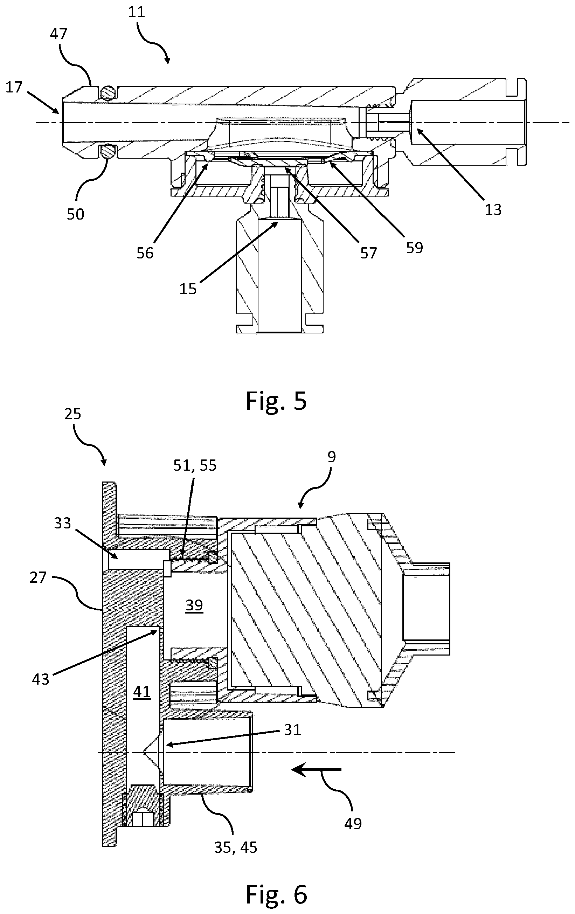

Wie in Fig. 6 gezeigt, kann zwischen dem zweiten Ende des Verbindungskanals 41 und der Atemgaskammer 39 optional eine Engstelle 43 mit einem deutlich kleineren Strömungsquerschnitt als der Verbindungskanal 41 angeordnet sein. Die Engstelle 43 kann beispielsweise als eine Art Düse fungieren.

In Fig. 2, Fig. 3 und Fig. 6 ist zudem eine optionale Ausführungsform der ersten Aufnahme 35 in Form einer Buchse 45 gezeigt. In die Buchse 45 kann ein die Austrittsöffnung 17 aufweisender Anschlussstutzen 47 der Koppeleinrichtung 11 (siehe Fig. 5) in einer Einführrichtung 49 (siehe Fig. 6) einführbar sein. Ein derartiges Stecker-Buchse-System ermöglicht eine unkomplizierte mechanische und fluidische Kopplung der Koppeleinrichtung 11 mit dem Grundkörper 27, insbesondere derart, dass die Koppeleinrichtung 11 luftdicht und/oder ausschließlich kraftschlüssig mit dem Grundkörper 27 verbunden ist.

Wie in Fig. 6 zu erkennen, kann die Buchse 45 einen sich in der Einführrichtung 49 geringfügig verjüngenden Querschnitt haben. Dies hat den Effekt, dass die Buchse 45 bzw. der Anschlussstutzen 47 beim Einführen des Anschlussstutzens 47 in die Buchse 45 mit einer radialen Kraft beaufschlagt wird, die umso größer wird, je tiefer der Anschlussstutzen 47 in die Buchse 45 eingeführt wird. Auf diese Weise kann bereits mit sehr geringem Kraftaufwand in der Einführrichtung 49 und auch bei größeren montage-, fertigungs- und/oder temperaturbedingten Maß- und/oder Formabweichungen eine ausreichende Dichtigkeit gewährleistet werden, ohne dass zusätzliche Maßnahmen erforderlich sind. Entsprechend einfach kann die Verbindung auch wieder gelöst werden.

Je nach Ausführungsform kann sich zusätzlich mindestens ein Dichtring um eine innere und/oder äußere Mantelfläche der Buchse 45 und/oder des Anschlussstutzens 47 erstrecken. Der Dichtring kann oder die Dichtringe können beim Einführen des Anschlussstutzens 47 in die Buchse 45 leicht zusammengedrückt werden, um eine luftdichte Verbindung zwischen der Buchse 45 und dem Anschlussstutzen 47 herzustellen.

In dem in Fig. 5 gezeigten Beispiel erstreckt sich ein solcher Dichtring 50 um eine äußere Mantelfläche des Anschlussstutzens 47.

Wie in Fig. 3 zu erkennen, kann die zweite Aufnahme 37 mehrere Gewinde zum Verschrauben des Grundkörpers 27 mit verschiedenen Typen des Gassensors 9 umfassen. In diesem Beispiel umfasst die zweite Aufnahme 37 ein zentrales Gewinde 51 zum Verschrauben des Grundkörpers 27 mit einem galvanischen Sauerstoffsensor und vier Schraubengewinde 53 zum Verschrauben des Grundkörpers 27 mit einem paramagnetischen Sauerstoffsensor (als Alternative zum galvanischen Sauerstoffsensor) über vier separate Schrauben 54 (siehe Fig. 2). Die Schraubengewinde 53 können gleichmäßig um das zentrale Gewinde 51 herum verteilt angeordnet sein. Die Gewinde 51, 53 können wie hier gezeigt als Innengewinde ausgeführt sein. Möglich sind aber auch Außengewinde.

Das zentrale Gewinde 51 kann einen deutlich größeren Gewindedurchmesser als jedes der Schraubengewinde 53 haben. Insbesondere kann das zentrale Gewinde 51 mit einem Gehäusegewinde 55 an einem zylinderförmigen Abschnitt eines Gehäuses des Gassensors 9 in Form des galvanischen Sauerstoffsensors verschraubbar sein (siehe Fig. 4).

Fig. 2 zeigt den Grundkörper 27 und den damit mittels mehrerer Schrauben 54 verschraubten Gassensor 9 in Form des paramagnetischen Sauerstoffsensors in perspektivischer Ansicht.

Fig. 6 zeigt den Grundkörper 27 und den damit verschraubten Gassensor 9 in Form des galvanischen Sauerstoffsensors aus Fig. 4 im Querschnitt. Wie hier zu erkennen, kann der das Gehäusegewinde 55 aufweisende zylinderförmige Abschnitt des Gehäuses des Gassensors 9, genauer dessen innere Mantelfläche, einen die Atemgaskammer 39 begrenzenden Abschnitt des Gassensors 9 bilden.

Fig. 5 zeigt einen Querschnitt durch die Koppeleinrichtung 11 aus Fig. 3. Die Koppeleinrichtung 11 kann so ausgebildet sein, dass sie sich abhängig von einer Druckdifferenz zwischen einem an der ersten Eintrittsöffnung 13 anliegenden ersten Atemgasdruck und einem an der zweiten Eintrittsöffnung 15 anliegenden zweiten Atemgasdruck zwischen der Kalibrierstellung und der Messstellung verstellt.

Beispielsweise kann sich die Koppeleinrichtung 11 in der Kalibrierstellung befinden, wenn der erste Atemgasdruck größer als der zweite Atemgasdruck oder gleich dem zweiten Atemgasdruck ist, und in der Messstellung befinden, wenn der erste Atemgasdruck kleiner als der zweite Atemgasdruck ist.

Um eine derartige druckabhängige Verstellung zu ermöglichen, kann die Koppeleinrichtung 11 eine spezielle Dichtmembran umfassen.

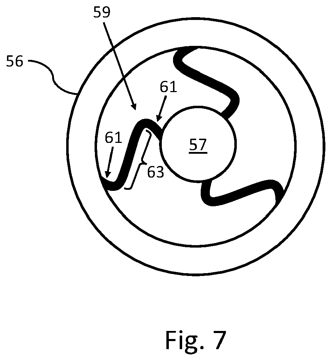

Fig. 7 zeigt eine mögliche Ausführungsform der Dichtmembran in der Draufsicht. Die Dichtmembran umfasst hier eine Basis 56 und ein mit der Basis 56 verbundenes und relativ zur Basis 56 auslenkbares Dichtelement 57. Das Dichtelement 57 kann mittels der Basis 56 derart in einem Strömungsweg des Atemgases durch die Koppeleinrichtung 11 positioniert sein, dass das Dichtelement 57 abhängig von der vorgenannten Druckdifferenz zwischen der Kalibrierstellung und der Messstellung relativ zur Basis 56 ausgelenkt werden kann. Beispielsweise kann die Kalibrierstellung einer Ruhestellung des Dichtelements 57 entsprechen, in der das Dichtelement 57 die zweite Eintrittsöffnung 15 luftdicht verschließt und somit einen Strömungsweg des Atemgases zwischen dem Patientenanschluss 7 und der Austrittsöffnung 17 versperrt. Durch Auslenken des Dichtelements 57 in die Messstellung, beispielsweise aufgrund des beim Ausatmen erzeugten Atemgasdrucks, kann die zweite Eintrittsöffnung 15 freigegeben werden.

Zweckmäßigerweise kann das Dichtelement 57 derart elastisch federnd ausgebildet sein und/oder derart elastisch federnd mit der Basis 56 verbunden sein, dass es in der Messstellung mit einer in Richtung der Kalibrierstellung wirkenden Rückstellkraft beaufschlagt wird.

Wie in Fig. 7 zu sehen, kann das Dichtelement 57 beispielsweise scheibenförmig ausgebildet sein und über mehrere, hier drei elastisch verformbare Federarme 59 elastisch federnd mit der Basis 56 verbunden sein, um die Auslenkung zwischen der Kalibrierstellung und der Messstellung zu ermöglichen. Dabei kann die Basis 56 ringförmig ausgebildet sein und konzentrisch zum Dichtelement 57 angeordnet sein. Das Dichtelement 57 kann dementsprechend in Richtung einer gemeinsamen Mittelachse der Basis 56 und des Dichtelements 57 relativ zur Basis 56 auslenkbar sein.

Es ist möglich, dass jeder der Federarme 59 zwei kürzere Längsabschnitte 61 und einen die zwei kürzeren Längsabschnitte 61 miteinander verbindenden längeren Längsabschnitt 63 umfasst, der deutlich länger als jeder der zwei kürzeren Längsabschnitte 61 oder als die zwei kürzeren Längsabschnitte 61 zusammen sein kann.

In dem in Fig. 7 gezeigten Beispiel ist jeder der Federarme 59 über seine zwei kürzeren Längsabschnitte 61 einerseits mit einem inneren Rand der Basis 56 und andererseits mit einem äußeren Rand des Dichtelements 57 verbunden.

Die zwei kürzeren Längsabschnitte 61 können in verschiedenen, beispielsweise einander entgegengesetzten Richtungen von den Enden des jeweiligen längeren Längsabschnitts 63 abstehen, wobei diese Richtungen jeweils von einer Längsrichtung des jeweiligen längeren Längsabschnitts 63 abweichen können.

Beispielsweise können die längeren Längsabschnitte 63 in ihrer jeweiligen Längsrichtung betrachtet zumindest teilweise gekrümmt verlaufen. Dies ermöglicht es, die Federarme 59 - bei einem gegebenen Abstand des äußeren Rands des Dichtelements 57 zum inneren Rand der Basis 56 in radialer oder axialer Richtung - möglichst lang und entsprechend biegsam auszuführen.

Abschließend wird darauf hingewiesen, dass Begriffe wie "aufweisen", "umfassen", "einschließe", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Beatmungsgerät
- 3: Atemgasanschluss
- 5: Atemgasquelle
- 7: Patientenanschluss
- 9: Gassensor
- 11: Koppeleinrichtung
- 13: erste Eintrittsöffnung
- 15: zweite Eintrittsöffnung
- 17: Austrittsöffnung
- 19: Gehäuse
- 21: Steuervorrichtung
- 23: Sensorsignal
- 25: Halterung
- 27: Grundkörper
- 29: Befestigungsabschnitt
- 31: Gaseinlass
- 33: Gasauslass
- 35: erste Aufnahme
- 37: zweite Aufnahme
- 39: Atemgaskammer
- 41: Verbindungskanal
- 43: Engstelle
- 45: Buchse
- 47: Anschlussstutzen
- 49: Einführrichtung
- 50: Dichtring
- 51: zentrales Gewinde
- 53: Schraubengewinde
- 54: Schraube
- 55: Gehäusegewinde
- 56: Basis
- 57: Dichtelement
- 59: Federarm
- 61: kürzerer Längsabschnitt
- 63: längerer Längsabschnitt

## Patentansprüche

1. Beatmungsgerät (1), umfassend:
einen Atemgasanschluss (3) zum Anschließen einer Atemgasquelle (5);
einen Patientenanschluss (7) zum Anschließen einer Patientenschnittstelle;
einen Gassensor (9) zum Erfassen einer Gaskonzentration;
eine Koppeleinrichtung (11), die eine mit dem Atemgasanschluss (3) verbundene erste Eintrittsöffnung (13), eine mit dem Patientenanschluss (7) verbundene zweite Eintrittsöffnung (15) und eine Austrittsöffnung (17) umfasst, wobei die Koppeleinrichtung (11) zwischen einer Kalibrierstellung und einer Messstellung verstellbar ist und ausgebildet ist, um die Austrittsöffnung (17) in der Kalibrierstellung nur mit der ersten Eintrittsöffnung (13) und in der Messstellung entweder nur mit der zweiten Eintrittsöffnung (15) oder sowohl mit der ersten Eintrittsöffnung (13) als auch mit der zweiten Eintrittsöffnung (15) fluidisch zu koppeln;
ein Gehäuse (19), das zumindest einen Teil der Komponenten des Beatmungsgeräts (1) umgibt, wobei die vom Gehäuse (19) umgebenen Komponenten des Beatmungsgeräts (1) den Gassensor (9) und/oder die Koppeleinrichtung (11) umfassen;
eine Halterung (25), die einen Grundkörper (27) mit einem Befestigungsabschnitt (29), einem Gaseinlass (31), einem Gasauslass (33), einer ersten Aufnahme (35) und einer zweiten Aufnahme (37) umfasst, wobei der Grundkörper (27) über den Befestigungsabschnitt (29) am und/oder im Gehäuse (19) befestigt ist, wobei die Koppeleinrichtung (11) von der ersten Aufnahme (35) aufgenommen ist, sodass die Austrittsöffnung (17) mit dem Gaseinlass (31) fluidisch gekoppelt ist, wobei der Gassensor (9) von der zweiten Aufnahme (37) aufgenommen ist, wobei der Grundkörper (27) zusammen mit dem Gassensor (9) eine Atemgaskammer (39) begrenzt, wobei die Atemgaskammer (39) einerseits mit dem Gaseinlass (31) und andererseits mit dem Gasauslass (33) verbunden ist, sodass Atemgas von der Austrittsöffnung (17) über den Gaseinlass (31) in die Atemgaskammer (39) und von der Atemgaskammer (39) über den Gasauslass (33) in eine Umgebung des Grundkörpers (27) strömen kann.

2. Beatmungsgerät (1) nach Anspruch 1,
wobei der Grundkörper (27) einen Teil einer Außenwand und/oder einer Innenwand des Gehäuses (19) bildet; und/oder
wobei das Gehäuse (19) eine Gehäuseöffnung zum Einführen des Gassensors (9) aufweist und der Grundkörper (27) die Gehäuseöffnung zumindest teilweise abdeckt.

3. Beatmungsgerät (1) nach Anspruch 2,
wobei die erste Aufnahme (35) und die zweite Aufnahme (37) an einer dem Inneren des Gehäuses (19) zugewandten ersten Seite des Grundkörpers (27) angeordnet sind und eine der ersten Seite gegenüberliegende zweite Seite des Grundkörpers (27) einen Teil der Außenwand des Gehäuses (19) bildet.

4. Beatmungsgerät (1) nach einem der vorhergehenden Ansprüche,
wobei die Koppeleinrichtung (11) abhängig von einer Druckdifferenz zwischen
einem an der ersten Eintrittsöffnung (13) anliegenden ersten Atemgasdruck und
einem an der zweiten Eintrittsöffnung (15) anliegenden zweiten Atemgasdruck zwischen der Kalibrierstellung und der Messstellung verstellbar ist.

5. Beatmungsgerät (1) nach Anspruch 4,
wobei sich die Koppeleinrichtung (11) in der Kalibrierstellung befindet, wenn der erste Atemgasdruck größer als der zweite Atemgasdruck oder gleich dem zweiten Atemgasdruck ist, und/oder in der Messstellung befindet, wenn der erste Atemgasdruck kleiner als der zweite Atemgasdruck ist.

6. Beatmungsgerät (1) nach Anspruch 4 oder 5,
wobei die Koppeleinrichtung (11) ferner eine Basis (56) und ein mit der Basis (56) verbundenes und relativ zur Basis (56) auslenkbares Dichtelement (57) umfasst, wobei das Dichtelement (57) mittels der Basis (56) derart in einem Strömungsweg des Atemgases durch die Koppeleinrichtung (11) positioniert ist, dass das Dichtelement (57) abhängig von der Druckdifferenz zwischen dem ersten Atemgasdruck und dem zweiten Atemgasdruck zwischen der Kalibrierstellung und der Messstellung relativ zur Basis (56) auslenkbar ist, wobei das Dichtelement (57) die zweite Eintrittsöffnung (15) in der Kalibrierstellung luftdicht verschließt und in der Messstellung freigibt.

7. Beatmungsgerät (1) nach Anspruch 6,
wobei das Dichtelement (57) derart elastisch federnd ausgebildet ist und/oder derart elastisch federnd mit der Basis (56) verbunden ist, dass es in der Messstellung mit einer in Richtung der Kalibrierstellung wirkenden Rückstellkraft beaufschlagt wird; und/oder
wobei das Dichtelement (57) über mindestens zwei elastisch verformbare Federarme (59) elastisch federnd mit der Basis (56) verbunden ist.

8. Beatmungsgerät (1) nach Anspruch 7,
wobei das Dichtelement (57) über die mindestens zwei Federarme (59) elastisch federnd mit der Basis (56) verbunden ist, wobei jeder der Federarme (59) zwei kürzere Längsabschnitte (61) und einen die zwei kürzeren Längsabschnitte (61) miteinander verbindenden längeren Längsabschnitt (63) umfasst, wobei der längere Längsabschnitt (63) länger als jeder der zwei kürzeren Längsabschnitte (61) oder länger als die zwei kürzeren Längsabschnitte (61) zusammen ist, wobei ein erster der zwei kürzeren Längsabschnitte (61) in einer von einer Längsrichtung des längeren Längsabschnitts (63) abweichenden ersten Richtung von einem ersten Ende des längeren Längsabschnitts (63) absteht und ein zweiter der zwei kürzeren Längsabschnitte (61) in einer von der ersten Richtung und/oder der Längsrichtung des längeren Längsabschnitts (63) abweichenden zweiten Richtung von einem zweiten Ende des längeren Längsabschnitts (63) absteht.

9. Beatmungsgerät (1) nach einem der Ansprüche 6 bis 8,
wobei die Basis (56) ringförmig ist; und/oder
wobei das Dichtelement (57) scheibenförmig ist.

10. Beatmungsgerät (1) nach einem der vorhergehenden Ansprüche,
wobei die erste Aufnahme (35) eine Buchse (45) zum Einführen eines die Austrittsöffnung (17) aufweisenden Anschlussstutzens (47) der Koppeleinrichtung (11) in einer Einführrichtung (49) umfasst.

11. Beatmungsgerät (1) nach Anspruch 10,
wobei die Buchse (45) einen sich in der Einführrichtung (49) verjüngenden Querschnitt hat.

12. Beatmungsgerät (1) nach einem der vorhergehenden Ansprüche,
wobei die zweite Aufnahme (37) ein Gewinde (51, 53) zum Verschrauben des Gassensors (9) mit dem Grundkörper (27) umfasst; oder
wobei die zweite Aufnahme (37) mindestens drei Gewinde (51, 53) zum Verschrauben des Gassensors (9) mit dem Grundkörper (27) umfasst.

13. Beatmungsgerät (1) nach einem der Ansprüche 1 bis 11,
wobei die zweite Aufnahme (37) mindestens drei Gewinde (51, 53) zum Verschrauben des Gassensors (9) mit dem Grundkörper (27) umfasst, wobei die Gewinde (51, 53) ein zentrales Gewinde (51) und mindestens zwei um das zentrale Gewinde (51) herum angeordnete Schraubengewinde (53) umfassen, wobei das zentrale Gewinde (51) ausgebildet ist, um ein Gehäusegewinde (55) an einem zylinderförmigen Abschnitt eines Gehäuses des Gassensors (9) aufzunehmen, wobei in jedes der Schraubengewinde (53) eine Schraube (54) schraubbar ist.

14. Beatmungsgerät (1) nach einem der vorhergehenden Ansprüche,
wobei der Grundkörper (27) ferner einen Verbindungskanal (41) umfasst, wobei der Gaseinlass (31) über den Verbindungskanal (41) mit der Atemgaskammer (39) verbunden ist, wobei zwischen dem Verbindungskanal (41) und der Atemgaskammer (39) eine Engstelle (43) mit einem im Vergleich zum Verbindungskanal (41) verringerten Strömungsquerschnitt angeordnet ist.

## Claims

1. A ventilator (1), comprising:
a breathing gas connection (3) for connecting a breathing gas source (5);
a patient connection (7) for connecting a patient interface;
a gas sensor (9) for detecting a gas concentration;
a coupling apparatus (11) comprising a first entry opening (13) connected to the breathing gas connection (3), a second entry opening (15) connected to the patient connection (7), and an exit opening (17), wherein the coupling apparatus (11) can be shifted between a calibration position and a measuring position and is designed to fluidically couple the exit opening (17), in the calibration position, only with the first entry opening (13) and, in the measuring position, either only with the second entry opening (15) or with both the first entry opening (13) and the second entry opening (15);
a housing (19) that surrounds at least some of the components of the ventilator (1), wherein the components of the ventilator (1) that are surrounded by the housing (19) include the gas sensor (9) and/or the coupling apparatus (11);
a holder (25) comprising a main body (27) with a fixing portion (29), a gas inlet (31), a gas outlet (33), a first receptacle (35), and a second receptacle (37), wherein the main body (27) is fixed to and/or in the housing (19) via the fixing portion (29), wherein the coupling apparatus (11) is received by the first receptacle (35) such that the exit opening (17) is fluidically coupled with the gas inlet (31), wherein the gas sensor (9) is received by the second receptacle (37), wherein the main body (27) together with the gas sensor (9) delimits a breathing gas chamber (39), wherein the breathing gas chamber (39) is connected on the one hand to the gas inlet (31) and on the other hand to the gas outlet (33) such that breathing gas can flow from the exit opening (17) via the gas inlet (31) into the breathing gas chamber (39) and from the breathing gas chamber (39) via the gas outlet (33) into the surroundings of the main body (27).

2. The ventilator (1) according to claim 1,
wherein the main body (27) forms a part of an outer wall and/or an inner wall of the housing (19); and/or
wherein the housing (19) has a housing opening for inserting the gas sensor (9), and the main body (27) at least partially covers the housing opening.

3. The ventilator (1) according to claim 2,
wherein the first receptacle (35) and the second receptacle (37) are arranged on a first side, which faces the inside of the housing (19), of the main body (27), and a second side, which is opposite to the first side, of the main body (27) forms a part of the outer wall of the housing (19).

4. The ventilator (1) according to one of the preceding claims,
wherein the coupling apparatus (11) can be shifted between the calibration position and the measuring position depending on a pressure difference between a first breathing gas pressure applied at the first entry opening (13) and a second breathing gas pressure applied at the second entry opening (15).

5. The ventilator (1) according to claim 4,
wherein the coupling apparatus (11) is located in the calibration position when the first breathing gas pressure is greater than the second breathing gas pressure or the same as the second breathing gas pressure, and/or is located in the measuring position when the first breathing gas pressure is less than the second breathing gas pressure.

6. The ventilator (1) according to claim 4 or 5,
wherein the coupling apparatus (11) also comprises a base (56) and a sealing element (57) which is connected to the base (56) and can be deflected relative to the base (56), wherein the sealing element (57) is positioned by means of the base (56) in a flow path of the breathing gas through the coupling apparatus (11) such that the sealing element (57) can be deflected relative to the base (56) between the calibration position and the measuring position depending on the pressure difference between the first breathing gas pressure and the second breathing gas pressure, wherein the sealing element (57) closes the second entry opening (15) in an air-tight manner in the calibration position and releases it in the measuring position.

7. The ventilator (1) according to claim 6,
wherein the sealing element (57) is designed to be elastically resilient and/or is connected to the base (56) in an elastically resilient manner such that a restoring force acting in the direction of the calibration position is applied to it in the measuring position; and/or
wherein the sealing element (57) is connected to the base (56) in an elastically resilient manner via at least two resiliently deformable spring arms (59).

8. The ventilator (1) according to claim 7,
wherein the sealing element (57) is connected to the base (56) in an elastically resilient manner via the at least two spring arms (59), wherein each of the spring arms (59) comprises two shorter longitudinal portions (61) and one longer longitudinal portion (63) connecting the two shorter longitudinal portions (61) to each other, wherein the longer longitudinal portion (63) is longer than each of the two shorter longitudinal portions (61) or longer than the two shorter longitudinal portions (61) together, wherein a first of the two shorter longitudinal portions (61) projects from a first end of the longer longitudinal portion (63) in a first direction that differs from a longitudinal direction of the longer longitudinal portion (63), and a second of the two shorter longitudinal portions (61) projects from a second end of the longer longitudinal portion (63) in a second direction that differs from the first direction and/or from the longitudinal direction of the longer longitudinal section (63).

9. The ventilator (1) according to one of claims 6 to 8,
wherein the base (56) is annular; and/or
wherein the sealing element (57) is disk-shaped.

10. The ventilator (1) according to one of the preceding claims,
wherein the first receptacle (35) comprises a socket (45) for inserting a connecting piece (47), which has the exit opening (17), of the coupling apparatus (11) in an insertion direction (49).

11. The ventilator (1) according to claim 10,
wherein the socket (45) has a cross-section that tapers in the insertion direction (49).

12. The ventilator (1) according to one of the preceding claims,
wherein the second receptacle (37) comprises a thread (51, 53) for screwing the gas sensor (9) to the main body (27); or
wherein the second receptacle (37) comprises at least three threads (51, 53) for screwing the gas sensor (9) to the main body (27).

13. The ventilator (1) according to one of claims 1 to 11,
wherein the second receptacle (37) comprises at least three threads (51, 53) for screwing the gas sensor (9) to the main body (27), wherein the threads (51, 53) comprise a central thread (51) and at least two screw threads (53) arranged around the central thread (51), wherein the central thread (51) is designed to receive a housing thread (55) on a cylindrical portion of a housing of the gas sensor (9), wherein a screw (54) can be screwed into each of the screw threads (53).

14. The ventilator (1) according to one of the preceding claims,
wherein the main body (27) also comprises a connecting channel (41), wherein the gas inlet (31) is connected to the breathing gas chamber (39) via the connecting channel (41), wherein a constriction (43) with a flow cross-section that is reduced compared to the connecting channel (41) is arranged between the connecting channel (41) and the breathing gas chamber (39).

## Revendications

1. Appareil respiratoire (1), comportant :
un raccord de gaz respiratoire (3) destiné à être raccordé à une source de gaz respiratoire (5) ;
un raccord de patient (7) destiné à être raccordé à une interface de patient ;
un capteur de gaz (9) destiné à détecter une concentration de gaz ;
un dispositif d'accouplement (11), lequel comporte une première ouverture d'entrée (13) reliée au raccord de gaz respiratoire (3), une deuxième ouverture d'entrée (15) reliée au raccord de patient (7) et une ouverture de sortie (17), dans lequel le dispositif d'accouplement (11) est réglable entre une position d'étalonnage et une position de mesure et conçu pour accoupler fluidiquement l'ouverture de sortie (17) uniquement à la première ouverture d'entrée (13) dans la position d'étalonnage et soit uniquement à la deuxième ouverture d'entrée (15), soit à la fois à la première ouverture d'entrée (13) et à la deuxième ouverture d'entrée (15) dans la position de mesure ;
un boîtier (19) entourant au moins une partie des composants de l'appareil respiratoire (1), dans lequel les composants de l'appareil respiratoire (1) entourés par le boîtier (19) comportent le capteur de gaz (9) et/ou le dispositif d'accouplement (11) ;
un support (25) comportant un corps de base (27) comprenant une section de fixation (29), une admission de gaz (31), une évacuation de gaz (33), un premier logement (35) et un deuxième logement (37), dans lequel le corps de base (27) est fixé à et/ou dans le boîtier (19) par le biais de la section de fixation (29), dans lequel le dispositif d'accouplement (11) est accueilli par le premier logement (35), de sorte que l'ouverture de sortie (17) est accouplée fluidiquement à l'admission de gaz (31), dans lequel le capteur de gaz (9) est accueilli par le deuxième logement (37), dans lequel le corps de base (27) délimite une chambre de gaz respiratoire (39) conjointement avec le capteur de gaz (9), dans lequel la chambre de gaz respiratoire (39) est reliée d'une part à l'admission de gaz (31) et d'autre part à l'évacuation de gaz (33), de sorte que du gaz respiratoire peut s'écouler à partir de l'ouverture de sortie (17) dans la chambre de gaz respiratoire (39) via l'admission de gaz (31) et de la chambre de gaz respiratoire (39) via l'évacuation de gaz (33) vers un environnement du corps de base (27).

2. Appareil respiratoire (1) selon la revendication 1,
dans lequel le corps de base (27) forme une partie d'une paroi extérieure et/ou d'une paroi intérieure du boîtier (19) ; et/ou
dans lequel le boîtier (19) présente une ouverture de boîtier pour l'introduction du capteur de gaz (9) et le corps de base (27) recouvre au moins partiellement l'ouverture de boîtier.

3. Appareil respiratoire (1) selon la revendication 2,
dans lequel le premier logement (35) et le deuxième logement (37) sont disposés sur un premier côté du corps de base (27) tourné vers l'intérieur du boîtier (19), et un deuxième côté du corps de base (27) opposé au premier côté forme une partie de la paroi extérieure du boîtier (19).

4. Appareil respiratoire (1) selon l'une des revendications précédentes,
dans lequel le dispositif d'accouplement (11) est réglable entre la position d'étalonnage et la position de mesure en fonction d'une différence de pression entre une première pression de gaz respiratoire appliquée à la première ouverture d'entrée (13) et une deuxième pression de gaz respiratoire appliquée à la deuxième ouverture d'entrée (15).

5. Appareil respiratoire (1) selon la revendication 4,
dans lequel le dispositif d'accouplement (11) se trouve dans la position d'étalonnage lorsque la première pression de gaz respiratoire est supérieure à la deuxième pression de gaz respiratoire ou égale à la deuxième pression de gaz respiratoire, et/ou se trouve dans la position de mesure lorsque la première pression de gaz respiratoire est inférieure à la deuxième pression de gaz respiratoire.

6. Appareil respiratoire (1) selon la revendication 4 ou 5,
dans lequel le dispositif d'accouplement (11) comporte en outre une base (56) et un élément d'étanchéité (57) relié à la base (56) et apte à être dévié par rapport à la base (56), dans lequel l'élément d'étanchéité (57) est positionné de telle façon au moyen de la base (56) dans un chemin d'écoulement du gaz respiratoire à travers le dispositif d'accouplement (11), que l'élément d'étanchéité (57) est déviable par rapport à la base (56) entre la position d'étalonnage et la position de mesure en fonction de la différence de pression entre la première pression de gaz respiratoire et la deuxième pression de gaz respiratoire, dans lequel l'élément d'étanchéité (57) ferme la deuxième ouverture d'entrée (15) de façon hermétique dans la position d'étalonnage et la libère dans la position de mesure.

7. Appareil respiratoire (1) selon la revendication 6,
dans lequel l'élément d'étanchéité (57) est conçu de façon élastique à effet ressort et/ou est relié de façon élastique à effet ressort à la base (56), de manière à être sollicité, dans la position de mesure, par une force de rappel agissant en direction de la position d'étalonnage ; et/ou
dans lequel l'élément d'étanchéité (57) est relié de façon élastique à effet ressort à la base (56) par le biais d'au moins deux bras de ressort (59) élastiquement déformables.

8. Appareil respiratoire (1) selon la revendication 7,
dans lequel l'élément d'étanchéité (57) est relié de façon élastique à effet ressort à la base (56) par le biais d'au moins deux bras de ressort (59), dans lequel chacun des bras de ressort (59) comporte deux sections longitudinales plus courtes (61) et une section longitudinale plus longue (63) reliant les deux sections longitudinales plus courtes (61) entre elles, dans lequel la section longitudinale plus longue (63) est plus longue que chacune des deux sections longitudinales plus courtes (61) ou plus longue que les deux sections longitudinales plus courtes (61) ensemble, dans lequel une première des deux sections longitudinales plus courtes (61) fait saillie à partir d'une première extrémité de la section longitudinale plus longue (63) dans une première direction différente d'une direction longitudinale de la section longitudinale plus longue (63), et une deuxième des deux sections longitudinales plus courtes (61) fait saillie à partir d'une deuxième extrémité de la section longitudinale plus longue (63) dans une deuxième direction différente de la première direction et/ou de la direction longitudinale de la section longitudinale plus longue (63).

9. Appareil respiratoire (1) selon l'une des revendications 6 à 8,
dans lequel la base (56) est annulaire ; et/ou
dans lequel l'élément d'étanchéité (57) est en forme de disque.

10. Appareil respiratoire (1) selon l'une des revendications précédentes, dans lequel le premier logement (35) comporte une douille (45) pour l'introduction d'une tubulure de raccordement (47) du dispositif d'accouplement (11) présentant l'ouverture de sortie (17) dans une direction d'introduction (49).

11. Appareil respiratoire (1) selon la revendication 10,
dans lequel la douille (45) présente une section transversale se rétrécissant dans la direction d'introduction (49).

12. Appareil respiratoire (1) selon l'une des revendications précédentes,
dans lequel le deuxième logement (37) comporte un filetage (51, 53) pour le vissage du capteur de gaz (9) avec le corps de base (27) ; ou
dans lequel le deuxième logement (37) comporte au moins trois filetages (51, 53) pour le vissage du capteur de gaz (9) avec le corps de base (27).

13. Appareil respiratoire (1) selon l'une des revendications 1 à 11,
dans lequel le deuxième logement (37) comporte au moins trois filetages (51, 53) pour le vissage du capteur de gaz (9) avec le corps de base (27), dans lequel les filetages (51, 53) comportent un filetage central (51) et au moins deux filetages de vis (53) disposés autour du filetage central (51), dans lequel le filetage central (51) est conçu pour recevoir un filetage de boîtier (55) au niveau d'une section cylindrique d'un boîtier du capteur de gaz (9), dans lequel une vis (54) peut être vissée dans chacun des filetages de vis (53).

14. Appareil respiratoire (1) selon l'une des revendications précédentes,
dans lequel le corps de base (27) comporte en outre un canal de liaison (41), dans lequel l'admission de gaz (31) est reliée à la chambre de gaz respiratoire (39) par le biais du canal de liaison (41), dans lequel un passage étroit (43) présentant une section transversale d'écoulement réduite en comparaison avec le canal de liaison (41) est disposé entre le canal de liaison (41) et la chambre de gaz respiratoire (39).
